(19) Europäisches Patentamt
European Patent Office
Office européen des brevets

(11) Publication number: 0 082 738 B1

(12) EUROPEAN PATENT SPECIFICATION

(45) Date of publication of patent specification: 22.03.89

(51) Int. Cl.[4]: C 07 D 211/44, C 07 D 227/08, C 08 K 5/34

(21) Application number: 82307054.5

(22) Date of filing: 22.12.82

# (54) Salts of amino organic peroxides and uses thereof.

(30) Priority: 23.12.81 IT 2582281

(43) Date of publication of application: 29.06.83 Bulletin 83/26

(45) Publication of the grant of the patent: 22.03.89 Bulletin 89/12

(84) Designated Contracting States: BE DE FR GB SE

(58) References cited:
EP-A-0 056 699
CH-A- 566 986

The file contains technical information submitted after the application was filed and not included in this specification

(73) Proprietor: AUSIMONT S.p.A.
31, Foro Buonaparte
I-20121 Milano (IT)

(72) Inventor: Fontana, Alberto
47, C.so Lodi
Milano (IT)
Inventor: Fontanelli, Renzo
19, Via Malocchi
Milan (IT)
Inventor: Sacrini, Egeo
9, Via Campiglio
Milan (IT)
Inventor: Cicchetti, Osvaldo
57, Via Sapri
Milano (IT)

(74) Representative: Lamb, John Baxter et al
MARKS & CLERK 57/60 Lincoln's Inn Fields
London WC2A 3LS (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 082 738 B1

## Description

This invention relates to salts of amino organic peroxides and, more particularly, to new organic peroxides having two peroxy functional groups and containing in their molecule an atom of salified aminic nitrogen, as well as to the use thereof in the cross-linking of plastomers, natural and synthetic elastomers and thermosetting resins.

It is known that organic peroxides are particularly important as generators of free radicals and, as a consequence, as starters of radical polymerizations, and as cross-linking agents for plastomers and vulcanizing agents for natural and synthetic elastomers.

However, not all peroxides are employable in cross-linking as some peroxides are very difficult to treat owing to their tendency to decompose, sometimes violently, at the temperature of use. Other peroxides have too short a semi-life time at the temperature of incorporation into the polymer, as a result of which they cause a certain degree of pre-cross-linking during the mixing step.

Netherlands Patent No. NL—A—137485 discloses 1,1-di(ter.butylperoxy)-3,3,5-trimethylcyclohexane which may be employed commercially with an inert inorganic carrier or with a plasticizer for example. Certain such compounds are sold commercially under the trade name TRIGONOX (Registeredl Trade Mark of AKZO Co.).

It is an object of the present invention to provide new peroxides having a good stability and a low volatility.

It has now been found that such an object may be achieved by using certain peroxides having two peroxy functional groups and containing in the molecule an atom of salified aminic nitrogen..

The present invention provides a new series of organic peroxides having two peroxy functional groups and containing in the molecule an atom of salified aminic nitrogen, having the general formula:

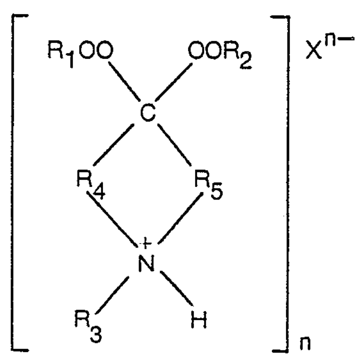

wherein:

$R_1$ and $R_2$ are each t.butyl, t.amyl, t.octyl or cumyl;

$R_3$ is hydrogen, an alkyl radical having 1 to 8 carbon atoms, a cycloalkyl radical having 5—6 carbon atoms in the ring, an aryl radical, or a benzyl radical;

$R_4$ is trimethylene and $R_5$ is methylene, or $R_4$ and $R_5$ are both ethylene, wherein $R_4$ and $R_5$ are in both the possibilities each at least monosubstituted by methyl or phenyl;

X is an anion of an organic or inorganic acid in which one or more acid hydrogens have been substituted by the aminic cation; and

n may be 1, 2 or 3 and represents the number of hydrogen atoms which are salified.

Some examples of compounds falling within the above indicated general formula are the following:

a) 4,4-di(t.butylperoxy)-2,2,6,6-tetramethylpiperidine formate,
b) 4,4-di(t.butylperoxy)-2,2,6,6-tetramethylpiperidine acetate,
c) 4,4-di(t.butylperoxy)-2,2,6,6-tetramethylpiperidine benzoate,
d) 4,4-di(t.butylperoxy)-2,2,6,6-tetramethylpiperidine oxalate,
e) 4,4-di(t.butylperoxy)-2,2,6,6-tetramethylpiperidine p-toluene sulphonate,
f) 4,4-di(t.butylperoxy)-2,2,6,6-tetramethylpiperidine acid sulphate,
g) 4,4-di(t.butylperoxy)-2,2,6,6-tetramethylpiperidine acid phosphate,
h) 4,4-di(t.butylperoxy)-2,2,6,6-tetramethylpiperidine hydrochloride,
i) 4,4-di(t.butylperoxy)-1,2,2,6,6-pentamethylpiperidine acid sulphate,
l) 4,4-di(t.butylperoxy)-1,2,2,6,6-pentamethylpiperidine acid phosphate,
m) 4,4-di(t.butylperoxy)-2,6-diphenylpiperidine acid sulphate,
n) 4,4-di(t.butylperoxy)-2,6-diphenylpiperidine formate,
o) 4,4-di(t.butylperoxy)-2,6-diphenylpiperidine benzoate,
p) 4,4-di(t.butylperoxy)-2,6-diphenylpiperidine acid phosphate,
q) 4,4-di(t.amylperoxy)-2,2,6,6-tetramethylpiperidine acid sulphate,
r) 4,4-di(t.amylperoxy)-2,2,6,6-tetramethylpiperidine acetate,
s) 4,4-di(cumylperoxy)-2,2,6,6-tetramethylpiperidine acid sulphate.

The salified amino diperoxides according to the present invention exhibit a relatively high and differentiated decomposition temperature depending on the type of acid whereby they have been salified; this allows the production, at moderate costs, of various peroxides derived from the same basic structure, operating in a relatively wide temperature range, so allowing the most suitable cross-linking and vulcanizing conditions to be selected.

Cross-linking is suitably accomplished at a temperature of from 100° to 200°C, more preferably from 140 to 170°C, at a pressure ranging from 49 to 196.2 bar (50 to 200 kg/cm$^2$), in time periods of 5—60 minutes, more preferably of 10—30 minutes. The weight concentration of the peroxide is suitably from 0.5 to 10%, more preferably from 2 to 6%, with respect to the plastomer.

Vulcanization is suitably carried out at a temperature of from 140° to 190°C, more preferably from 150° to 170°C, in time periods of 5—200 minutes, more preferably of 5—30 minutes. The peroxide weight concentration is suitably from 0.5 to 10%, more preferably from 2 to 6%, with respect to the elastomer.

Mixtures having the following composition have proved particularly suitable:

| | | |
|---|---|---|
| Ethylene-propylene copolymer | 100 | parts by weight |
| HAF carbon black | 20—80 | " " " |
| ZnO (or MgO) | 1—10 | " " " |
| Sulphur | 0.1—0.5 | " " " |
| Peroxide | 0.005—0.02 | moles. |

(HAF carbon black is High Abrasion Furnace carbon black, according to ASTM No. 330).

More particularly, a composition having the following essential components, other than the peroxide, may be utilized:

| | | |
|---|---|---|
| ethylene/propylene copolymer | 100 | parts by weight |
| carbon black | 50 | " " " |
| ZnO (or MgO) | 5 | " " " |
| Sulphur | 0.3 | " " " |

The hardening of a thermosetting resin, particularly an unsaturated polyester resin, is suitably accomplished at a temperature of from 50° to 170°C, more preferably from 80 to 115°C, or alternatively more preferably from 100 to 130°C. The peroxide concentration is suitably from 0.01 to 5% by weight.

The diperoxides of the present invention are obtainable according to various methods. According to a preferred method, the diperoxides of this invention can be prepared by reacting an aminic organic diperoxide (described in co-pending Italian patent application No. 19,165 A/81 in the name of the present Applicant) with an organic or inorganic acid.

The invention will be further described with reference to the following illustrative Examples.

## Example 1

Preparation of 4,4-di(t.butylperoxy)-2,2,6,6-tetramethylpiperidine formate.

31.7 g (0.1 moles) of 4,4-di(t.butylperoxy)-2,2,6,6-tetramethylpiperidine in 100 cc of hexane were introduced into a glass flask and 4.9 g (0.09 moles) of 85% formic acid were dropped thereinto under stirring at room temperature. The resulting precipitate was filtrated and washed with hexane.

After drying, 32 g of a white crystalline product were obtained, which, on the basis of the centesimal analysis

C: 59.31% found (59.47% calculated)
H: 10.27% found (10.26% calculated)
N: 3.91% found ( 3.85% calculated)

was identified as 4,4-di(t.butylperoxy)-2,2,6,6-tetramethylpiperidine formate having the following formula:

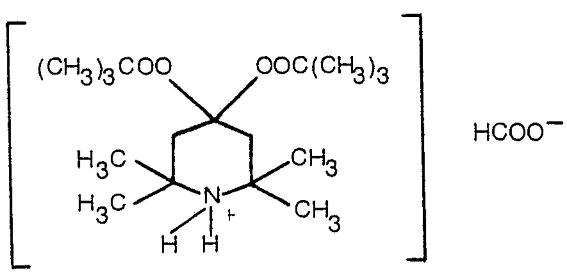

The melting and decomposition temperature of the product in a glass capillary was 52°C.

Example 2

Preparation of 4,4-di(t.butylperoxy)-2,2,6,6-tetramethylpiperidine acetate.

The method was as described in Example 1 and 5.4 g (0.09 moles) of glacial acetic acid were dropped under stirring at room temperature.

The resulting precipitate was filtered and washed with hexane; after drying, 33.5 g of a white crystalline product were obtained which, on the basis of the centesimal analysis

C: 60.31% found (60.45% calculated)
H: 10.41% found (10.41% calculated)
N: 3.62% found ( 3.71% calculated)

was identified as 4,4-di(t.butylperoxy)-2,2,6,6-tetramethylpiperidine acetate of formula:

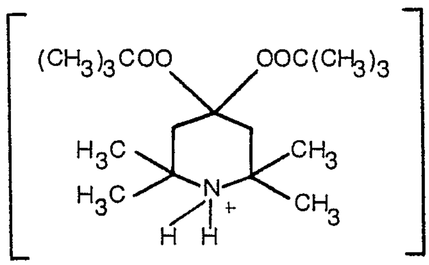
$CH_3COO^-$

The melting and decomposition temperature of the product in a glass capillary was 149°C.

Example 3

Preparation of 4,4-di(t.butylperoxy)-2,2,6,6-tetramethylpiperidine benzoate.

By operating according to Example 1, a solution of 11 g (0.09 moles) of benzoic acid in 80 cc of ethyl ether was dropped under stirring at room temperature. The resulting precipitate was filtered and washed with hexane.

After drying, 38.5 g of a white crystalline product were obtained, which, on the basis of the centesimal analysis

C: 65.44% found (65.57% calculated)
H: 9.40% found ( 9.40% calculated)
N: 3.11% found ( 3.18% calculated)

and of I.R. analysis was identified as 4,4-di(t.butylperoxy)-2,2,6,6-tetramethylpiperidine benzoate having the formula:

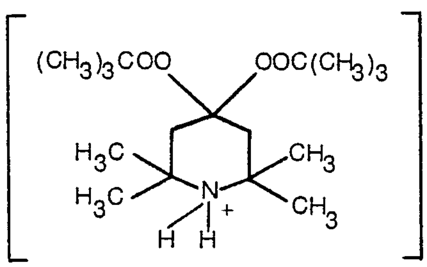
$C_6H_5COO^-$

The melting and decomposition temperature of the product in a glass capillary was 178°C.

Example 4

Preparation of 4,4-di(t.butylperoxy)-2,2,6,6-tetramethylpiperidine oxalate.

Into a glass flask there were introduced 31.7 g (0.1 moles) of 4,4-di(t.butylperoxy)-2,2,6,6-tetramethylpiperidine in 100 cc of hexane: a solution of 5.7 g (0.045 moles) of dihydrated oxalic acid in 20 cc of methanol was then dropped thereinto under stirring and at room temperature. The precipitate so obtained was filtrated and washed with methanol. After drying, 20 g of a white crystalline product were obtained, which, on the basis of the centesimal analysis

C: 59.58% found (59.64% calculated)
H: 10.06% found (10.01% calculated)
N: 3.80% found ( 3.86% calculated)

was identified as 4,4-di(t.butylperoxy)-2,2,6,6-tetramethylpiperidine oxalate of formula:

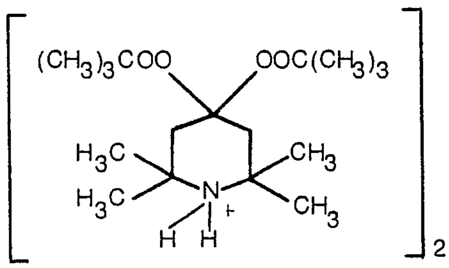

$C_2O_4^{--}$

The melting and decomposition temperature of the product in a glass capillary was 155°C.

Example 5

Preparation of 4,4-di(t.butylperoxy)-2,2,6,6-tetramethylpiperidine p-toluene sulphonate.

By operating as described in the preceding examples, a solution of 17 g (0.09 moles) of monohydrated p-toluenesulphonic acid in 5 g of water was dropped under stirring at room temperature into a glass flask containing 31.7 g (0.1 mols) of 4,4-di(t.butylperoxy)-2,2,6,6-tetramethylpiperidine in 100 cc of hexane. The resulting precipitate was filtered and washed with 5 cc of water and successively with hexane. After drying, 43 g of white crystalline product were obtained, which, on the basis of the centesimal analysis

| | | |
|---|---|---|
| C: | 58.75% found | (58.87% calculated) |
| H: | 8.79% found | ( 8.85% calculated) |
| N: | 2.89% found | ( 2.86% calculated) |
| S: | 6.49% found | ( 6.54% calculated) |

and of I.R. analysis, was identified as 4,4-di(t.butylperoxy)-2,2,6,6-tetramethylpiperidine p-toluene-sulphonate having the formula:

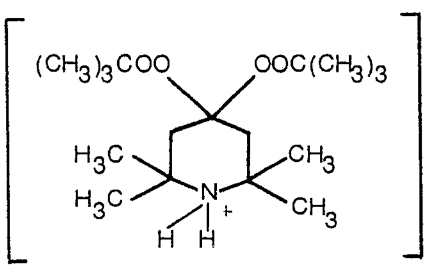

$CH_3-C_6H_4-SO_3^-$

The melting and decomposition temperature of the product in a glass capillary was 167°C.

Example 6

Preparation of 4,4-di(t.butylperoxy)-2,2,6,6-tetramethylpiperidine acid sulphate.

By operating as described in the preceding examples, 24.4 g of an aqueous solution at 40% of sulphuric acid (0.1 moles) were dropped under stirring at room temperature into a glass flask containing 31.7 g (0.1 mols) of 4,4-di(t.butylperoxy)-2,2,6,6-tetramethylpiperidine in 100 cc of hexane. The resulting precipitate was filtered and washed with 5 cc of cold water and then with hexane.

After drying, 40 g of a white crystalline product were obtained, which, on the basis of the centesimal analysis

| | | |
|---|---|---|
| C: | 49.44% found | (49.14% calculated) |
| H: | 9.01% found | ( 8.97% calculated) |
| N: | 3.31% found | ( 3.37% calculated) |
| S: | 7.66% found | ( 7.71% calculated) |

was identified as 4,4-di(t.butylperoxy)-2,2,6,6-tetramethylpiperidine acid sulphate of formula:

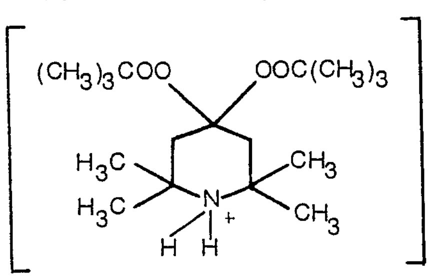

$HSO_4^-$

The melting and decomposition temperature of the product in a glass capillary was 153°C.

Example 7

Preparation of 4,4-di(t.butylperoxy)-2,2,6,6-tetramethylpiperidine acid phosphate.

10.4 g (0.09 moles) of 85% phosphoric acid were dropped under stirring at room temperature into a glass flask containing 31.7 g (0.1 moles) of 4,4-di(t.butylperoxy)-2,2,6,6-tetramethylpiperidine in 100 cc of hexane, as described in the preceding examples.

The precipitate so obtained was filtered and washed with 5 cc of water, then with hexane. After drying there were obtained 36.5 g of a white crystalline product which, on the basis of the centesimal analysis

| | | |
|---|---|---|
| C: | 48.98% found | (49.14% calculated) |
| H: | 9.15% found | ( 9.22% calculated) |
| N: | 3.42% found | ( 3.37% calculated) |
| S: | 7.39% found | ( 7.45% calculated) |

was identified as 4,4-di(t.butylperoxy)-2,2,6,6-tetramethylpiperidine acid phosphate having the formula:

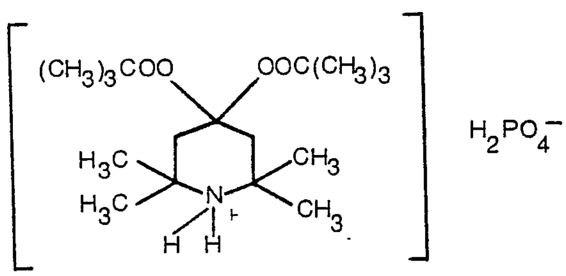

The melting and decomposition temperature of the product in a glass capillary was 172°C.

Example 8

Preparation of 4,4-di(t.butylperoxy)-2,2,6,6-tetramethylpiperidine hydrochloride.

31.7 g (0.1 moles) of 4,4-di(t.butylperoxy)-2,2,6,6-tetramethylpiperidine in 100 cc of hexane were introduced into a glass flask: 13.4 g of an aqueous solution at 25% of hydrochloric acid (0.09 moles) were then dropped thereinto under stirring, at room temperature. The resulting precipitate was filtered and washed with 5 cc of cold water and successively with hexane.

After drying, 31.5 g of a white crystalline product were obtained, which on the basis of the centesimal analysis

| | | |
|---|---|---|
| C: | 57.77% found | (57.68% calculated) |
| H: | 10.29% found | (10.25% calculated) |
| N: | 3.89% found | ( 3.96% calculated) |
| Cl: | 10.04% found | (10.01% calculated) |

was identified as 4,4-di(t.butylperoxy)-2,2,6,6-tetramethylpiperidine hydrochloride of formula:

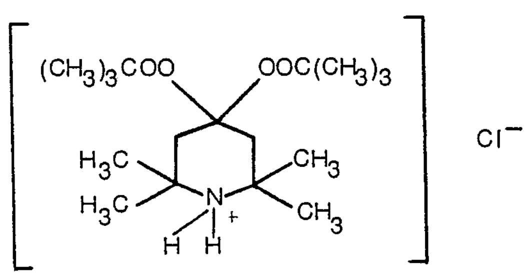

The melting and decomposition temperature of this product in a glass capillary was 170°C.

Example 9

Preparation of 4,4-di(t.butylperoxy)-1,2,2,6,6-pentamethylpiperidine acid sulphate.

Into a glass flask there were introduced 34.9 g (0.1 moles) of 4,4-di(t.butylperoxy)-1,2,2,6,6-pentamethylpiperidine having a titre of 95% in 100 cc of hexane: successively, 24.4 g of an aqueous solution at 40% of sulphuric acid (0.1 moles) were dropped therein, under stirring, at room temperature. The resulting precipitate was filtered and washed with 5 cc of cold water and successively with hexane. After drying, 42 g of a white crystalline product were obtained; such product was identified, on the basis of the centesimal analysis

| | | |
|---|---|---|
| C: | 50.29% found | (50.34% calculated) |
| H: | 9.13% found | ( 9.15% calculated) |
| N: | 3.22% found | ( 3.26% calculated) |
| S: | 7.15% found | ( 7.46% calculated) |

as 4,4-di(t.butylperoxy)-1,2,2,6,6-pentamethylpiperidine acid sulphate having the formula:

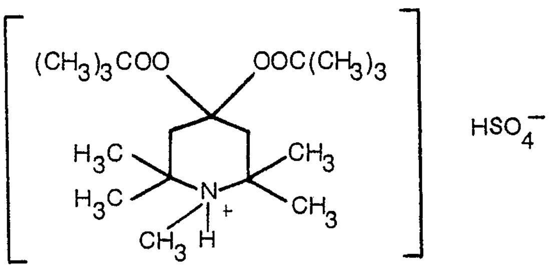

The melting temperature of this product in a glass capillary was 80°C and the decomposition temperature was 100°C.

Example 10

Crosslinking

The cross-linking tests were carried out on mixes based on polyethylene such as Fertene UK5—1830 (registered Trade Mark of Montedison S.p.A.) having a density of 0.917 and a grade of 1.2 g/10 min, at 190°C (ASTM D 1238), additioned with 0.01 moles of peroxide per 100 g of polyethylene.

The following Table 1 indicates the conditions under which the cross-linking was carried out and the cross-linking degree obtained by using the peroxide prepared according to Example 6 in comparision with 4,4-di(t.butylperoxy)-2,2,6,6-tetramethylpiperidine. The polyethylene cross-linking degree was measured by determining the solubility of the polymer in an organic solvent (decalin), according to the procedure fixed by method ASTM D 2765. The non cross-linked polyethylene was completely soluble in the solvent.

TABLE 1

Cross-Linking

| Formulation | | X* | A** | in the absence of peroxide |
|---|---|---|---|---|
| low density polyethylene<br>peroxide moles | parts b. wght. | 100<br>0.01 | 100<br>0.01 | 100<br>— |
| cross-linking in a press | | 30 min. at 150°C | 30 min. at 165°C | — |
| undissolved polyethylene | g/100 g | 66.2 | 60 | fully soluble |

Note:
X* 4,4-di-t.butylperoxy-2,2,6,6-tetramethylpiperidine
A** 4,4-di-t.butylperoxy-2,2,6,6-tetramethylpiperidine acid sulphate.

Example 11

Vulcanization

The vulcanization tests were carried out on mixes based on ethylene-propylene copolymer DUTRAL CO 054 (registered Trade Mark of Montedison S.p.A.) consisting of 55 mol-% of ethylene — Mooney viscosity ML(1+4) at 100°C=45. Table 2 indicates the conditions under which vulcanization was carried out and the rheological and physical characteristics of the vulcanizates obtained by employing 4,4-di-(t.butylperoxy)-2,2,6,6-tetramethylpiperidine acid sulphate, prepared in example 6, in comparison with 4,4-di-(t.butylperoxy)-2,2,6,6-tetramethylpiperidine.

TABLE 2

| Formulation | | X | A |
|---|---|---|---|
| Dutral CO 054 | parts by weight | 100 | 100 |
| Carbon black HAF | " " " | 50 | 50 |
| ZnO | " " " | 5 | 5 |
| S | " " " | 0.3 | 0.3 |
| moles of peroxide | | 0.01 | 0.01 |
| Vulcanization in press | | 20′ at 150°C | 30′ at 165°C |
| Initial characteristics | | | |
| Tensile strength | bar | 200.1 | 186.4 |
| Elongation at break | % | 293 | 315 |
| 200% modulus | bar | 104 | 88.3 |
| Tear strength | kg/cm | 37 | 40 |
| Shore A hardness | | 70 | 70 |
| Characteristics after ageing in oven (7 days at 125°C) | | | |
| tensile strength % | | −3.6 | +14.8 |
| elongation at break % | | +12 | +14.4 |
| 200% modulus | | −12 | −5 |
| Shore A hardness (variation in points) | | +1 | +1 |
| Compression set % (70 h at 100°C) | | 15 | 25 |
| Mooney scorth ($t_{10}$) min. | | 6′ 45″ | |

X=4,4-di(t.butylperoxy)-2,2,6,6-tetramethylpiperidine
A=4,4-di(t.butylperoxy)-2,2,6,6-tetramethylpiperidine acid sulphate.

Example 12

Hardening of unsaturated polyester resins.

The hardening tests of unsaturated polyester resins were carried out on a resin having the following composition:

| | | |
|---|---|---|
| phthalic anhydride | 0.6 | moles |
| maleic anhydride | 0.4 | moles |
| propylene glycol | 1.05 | moles |
| hydroquinone | 150 | ppm |
| styrene | 36% | referred to the finished product. |

Such resin, additioned with 1% of peroxide, was hardened at a temperature of 100°C and 130°C.

Table 3 shows the time required to obtain hardening of the resin for the following peroxides:

A: 4,4-di(t.butylperoxy)-2,2,6,6-tetramethylpiperidine acid phosphate
B: 4,4-di(t.butylperoxy)-2,2,6,6-tetramethylpiperidine p.toluenesulphonate
C: 4,4-di(t.butylperoxy)-2,2,6,6-tetramethylpiperidine oxalate
D: 4,4-di(t.butylperoxy)-2,2,6,6-tetramethylpiperidine benzoate
E: 4,4-di(t.butylperoxy)-2,2,6,6-tetramethylpiperidine acetate
F: 4,4-di(t.butylperoxy)-2,2,6,6-tetramethylpiperidine formate
G: 4,4-di(t.butylperoxy)-2,2,6,6-tetramethylpiperidine acid sulphate
as compared with the peroxide
X: 4,4-di(t.butylperoxy)-2,2,6,6-tetramethylpiperidine.

TABLE 3

Hardening of unsaturated polyester resins

| peroxide | hardening time at 130°C | hardening time at 100°C |
|---|---|---|
| A | 5.5 min. | 52 min. |
| B | 5 min. | 40 min. |
| C | 5 min. | n. d. |
| D | 5 min. | 35 min. |
| E | 4.7 min. | 36 min. |
| F | 4.5 min. | 28 min. |
| G | n. d. | 49 min. |
| X | 5 min. | 21 min. |

n. d.: not determined

**Claims**

1. An organic peroxide characterised by having two peroxy functional groups and containing in the molecule an atom of salified aminic nitrogen, having the general formula:

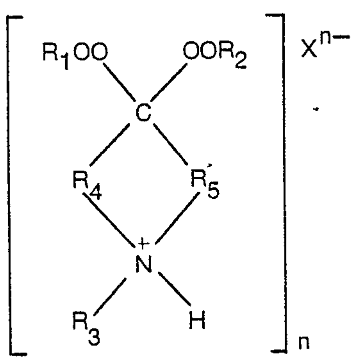

wherein:

$R_1$ and $R_2$ are each t.butyl, t-amyl, t-octyl or cumyl;

$R_3$ is hydrogen, an alkyl radical having 1 to 8 carbon atoms, a cycloalkyl radical having 5—6 carbon atoms in the ring, an aryl radical, or a benzyl radical;

$R_4$ is trimethylene and $R_5$ is methylene, or $R_4$ and $R_5$ are both ethylene, wherein $R_4$ and $R_5$ are in both the possibilities each at least monosubstituted by methyl or phenyl;

X is an anion of an inorganic or organic acid in which one or more acid hydrogens have been substituted by the aminic cation; and

n may be 1, 2 or 3 and represents the number of hydrogen atoms which are salified.

2. 4,4-di(t.butylperoxy)-2,2,6,6-tetramethylpiperidine formate.

3. 4,4-di(t.butylperoxy)-2,2,6,6-tetramethylpiperidine acetate.

4. 4,4-di(t.butylperoxy)-2,2,6,6-tetramethylpiperidine benzoate.

5. 4,4-di(t.butylperoxy)-2,2,6,6-tetramethylpiperidine oxalate.

6. 4,4-di(t.butylperoxy)-2,2,6,6-tetramethylpiperidine p-toluene sulphonate.

7. 4,4-di(t.butylperoxy)-2,2,6,6-tetramethylpiperidine acid sulphate.

8. 4,4-di(t.butylperoxy)-2,2,6,6-tetramethylpiperidine acid phosphate.

9. 4,4-di(t.butylperoxy)-2,2,6,6-tetramethylpiperidine hydrochloride.

10. 4,4-di(t.butylperoxy)-1,2,2,6,6-pentamethylpiperidine acid sulphate.

11. A composition characterised by comprising a plastomeric or elastomeric natural or synthetic polymer and a compound as claimed in any of claims 1 to 10.

12. A composition as claimed in claim 11, characterised in that the plastomeric polymer is polyethylene or the elastomeric polymer is an ethylene-propylene copolymer.

13. A composition as claimed in claim 11 or 12, characterised in that the said peroxide is utilized as a cross-linking agent in an amount of from 0.5 to 10% referred to the plastomer.

14. A composition as claimed in claim 13, characterised in that the amount of peroxide as cross-linking agent is from 2 to 6% referred to the plastomer.

15. A composition as claimed in claim 11 or 12, characterised in that the said peroxide is utilized as a vulcanizing agent in an amount of from 0.5 to 10% referred to the elastomer.

16. A composition as claimed in claim 15, characterised in that the amount of peroxide as vulcanizing agent is from 2 to 6% referred to the elastomer.

17. A composition as claimed in claim 12, characterised by further comprising a filler and a cross-linking co-agent.

18. A composition as claimed in claim 12, characterised in that the components thereof, other than the said peroxide, are present in the following ratios:

| | |
|---|---|
| ethylene/propylene copolymer | 100 parts by weight |
| carbon black | 50 parts by weight |
| ZnO (or MgO) | 5 parts by weight |
| Sulphur | 0.3 parts by weight |

19. A composition characterised by comprising a thermosetting resin and a compound as claimed in any of claims 1 to 10.

20. A composition as claimed in claim 19, characterised in that the thermosetting resin is an unsaturated polyester resin.

21. A composition as claimed in claim 20, characterised in that the said peroxide is utilized as a hardening agent in an amount of from 0.01 to 5% referred to the unsaturated polyester resin.

22. A process as claimed in claim 20, characterised by further comprising a filler and a cross-linking co-agent.

23. A process for cross-linking the composition according to claim 11, 12 or 13, characterised in that cross-linking is effected at a temperature of from 100° to 200°C, at a pressure of from 49 to 196.2 bar (50 to 200 kg/cm$^2$), for 5—60 minutes.

24. A process as claimed in claim 23, characterised in that the said temperature is from 140° to 170°C and the said time is 10—30 minutes.

25. A process for vulcanizing the composition according to claim 11, 12 or 15, characterised in that vulcanizing is effected at a temperature of from 140° to 190°C, for 5—200 minutes.

26. A process as claimed in claim 25, characterised in that the said temperature is 150° to 170°C and the said time is 5—30 minutes.

27. A process for hardening the composition according to claim 19, 20 or 21, characterised in that hardening is effected at a temperature of from 50° to 170°C.

28. A process as claimed in claim 27, characterised in that the said temperature is from 80° to 115°C.

29. The use of the diperoxides according to any of claims 1 to 10 as cross-linking agents for plastomers, natural or synthetic elastomers and unsaturated polyester resins.

**Patentansprüche**

1. Organisches Peroxid, dadurch gekennzeichnet, daß es zwei funktionelle Peroxygruppen aufweist und im Molekül ein in ein Salz umgewandeltes Amin-Stickstoffatom enthält, mit der folgenden Formel:

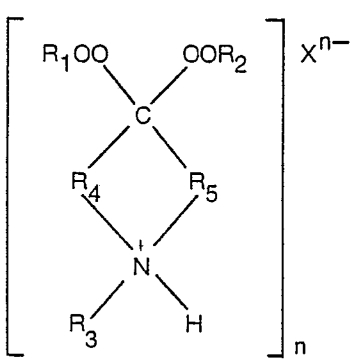

in welcher:

$R_1$ und $R_2$ jeweils tert.-Butyl, tert.-Amyl, tert.-Octyl oder tert.-Cumyl ist;

$R_3$ für Wasserstoff, einen Alkylrest mit 1 bis 8 Kohlenstoffatomen, einen Cycloalkylrest mit 5 bis 6 Kohlenstoffatomen im Ring, einen Arylrest oder einen Benzylrest steht;

$R_4$ Trimethylen und $R_5$ Methylen bedeuten oder $R_4$ und $R_5$ beide Ethylen sind, wobei $R_4$ und $R_5$ in beiden Fällen wenigstens monosubstituiert durch Methyl oder Phenyl sind;

X ein Anion einer anorganischen und organischen Säure ist, in welcher ein oder mehrere saure Wasserstoffe durch das Amin-Kation ersetzt worden sind; und

n 1, 2 oder 3 sein kann und die Zahl der Wasserstoffatome wiedergibt, die in ein Salz umgewandelt wurden.

2. 4,4-Di(tert.-butylperoxy)-2,2,6,6-tetramethylpiperidinformiat.

3. 4,4-Di(tert.-butylperoxy)-2,2,6,6-tetramethylpiperidinacetat.

4. 4,4-Di(tert.-butylperoxy)-2,2,6,6-tetramethylpiperidinbenzoat.

5. 4,4-Di(tert.-butylperoxy)-2,2,6,6-tetramethylpiperidinoxalat.

6. 4,4-Di(tert.-butylperoxy)-2,2,6,6-tetramethylpiperidin-p-toluolsulphonat.

7. Saures 4,4-Di(tert.-butylperoxy)-2,2,6,6-tetramethylpiperidinsulphat.

8. Saures 4,4-Di(tert.-butylperoxy)-2,2,6,6-tetramethylpiperidinphosphat.

9. 4,4-Di(tert.-butylperoxy)-2,2,6,6-tetramethylpiperidinhydrochlorid.

10. Saures 4,4-Di(tert.-butylperoxy)-1,2,2,6,6-pentamethylpiperidinsulphat.

11. Zusammensetzung, dadurch gekennzeichnet, daß sie ein natürliches oder synthetisches plastomeres oder elastomeres Polymeres und eine Verbindung gemäß einem der Ansprüche 1 bis 10 umfaßt.

12. Zusammensetzung nach Anspruch 11, dadurch gekennzeichnet, daß das plastomere Polymere Polyethylen oder das elastomere Polymere ein Ethylen-Propylen-Copolymeres ist.

13. Zusammensetzung nach Anspruch 11 oder 12, dadurch gekennzeichnet, daß das Peroxid als Vernetzungsmittel in einer Menge von 0,5 bis 10%, bezogen auf das Plastomere, verwendet wird.

14. Zusammensetzung nach Anspruch 13, dadurch gekennzeichnet, daß die Menge an Peroxid als Vernetzungsmittel von 2 bis 6%, bezogen auf das Plastomere, beträgt.

15. Zusammensetzung nach Anspruch 11 oder 12, dadurch gekennzeichnet, daß das Peroxid als Vulkanisiermittel in einer Menge von 0,5 bis 10%, bezogen auf das Elastomere, verwendet wird.

16. Zusammensetzung nach Anspruch 15, dadurch gekennzeichnet, daß die Menge an Peroxid als Vulkanisiermittel 2 bis 6%, bezogen auf das Elastomere, beträgt.

17. Zusammensetzung nach Anspruch 12, dadurch gekennzeichnet, daß sie weiter einen Füllstoff und ein Co-Vernetzungsmittel umfaßt.

18. Zusammensetzung nach Anspruch 12, dadurch gekennzeichnet, daß die vom Peroxid verschiedenen Komponenten derselben in den folgenden Verhältnissen anwesend sind:

| | |
|---|---|
| Ethylen/Propylen-Copolymeres | 100 Gewichtsteile |
| Ruß | 50 Gewichtsteile |
| ZnO (oder MgO) | 5 Gewichtsteile |
| Schwefel | 0,3 Gewichtsteile. |

19. Zusammensetzung, dadurch gekennzeichnet, daß sie ein hitzehärtbares Harz und eine Verbindung nach einem der Ansprüche 1 bis 10 umfaßt.

20. Zusammensetzung nach Anspruch 19, dadurch gekennzeichnet, daß das hitzehärtbare Harz ein ungesättigtes Polyesterharz ist.

21. Zusammensetzung nach Anspruch 20, dadurch gekennzeichnet, daß das Peroxid als Härtungsmittel in einer Menge von 0,01 bis 5%, bezogen auf das ungesättigte Polyesterharz, verwendet wird.

22. Zusammensetzung nach Anspruch 20, dadurch gekennzeichnet, da sie weiter einen Füllstoff und ein Co-Vernetzungsmittel umfaßt.

23. Verfharen zur Vernetzung der Zusammensetzungen nach den Ansprüchen 11, 12 oder 13, dadurch gekennzeichnet, daß die Vernetzung bei einer Temperatur von 100 bis 200°C, einem Druck von 49 bis 196,2 bar (50 bis 200 kg/cm$^2$) für 5 bis 60 Minuten bewirkt wird.

24. Verfahren nach Anspruch 23, dadurch gekennzeichnet, daß die Temperatur 140 bis 170°C und die Zeit 10 bis 30 Minuten beträgt.

25. Verfahren zur Vulkanisierung der Zusammensetzungen nach Anspruch 11, 12 oder 15, dadurch gekennzeichnet, daß die Vulkanisierung bei einer Temperatur von 140 bis 190°C für 5 bis 200 Minuten bewirkt wird.

26. Verfahren nach Anspruch 25, dadurch gekennzeichnet, daß die Temperatur 150 bis 170°C und die Zeit 5 bis 30 Minuten beträgt.

27. Verfahren zur Härtung der Zusammensetzung nach Anspruch 19, 20 oder 21, dadurch

gekennzeichnet, daß die Härtung bei einer Temperatur von 50 bis 170°C bewirkt wird.

28. Verfahren nach Anspruch 27, dadurch gekennzeichnet, daß die Temperatur 80 bis 115°C beträgt.

29. Verwendung der Diperoxide gemäß irgendeinem der Ansprüche 1 bis 10 als Vernetzungsmittel für Plastomere, natürliche oder synthetische Elastomere und ungesättigte Polyesterharze.

**Revendications**

1. Un peroxyde organique caractérisé en ce qu'il présente deux groupes fonctionnels peroxy et contient dans la molécule un atome d'azote aminé salifié, présentant la formule générale suivante:

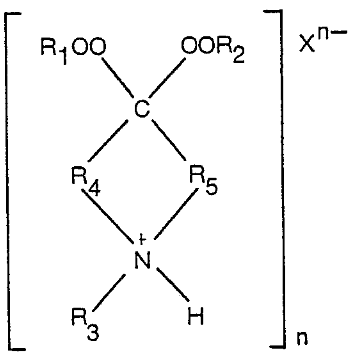

dans laquelle:

$R_1$ et $R_2$ consistent en t.butyle, t.amyle, t.octyle ou cumyle;

$R_3$ est un atome d'hydrogène, un radical alkyle comprenant de 1 à 8 atomes de carbone, un radical cycloalkyle comprenant de 5 à 6 atomes de carbone dans le cycle, un radical aryle ou un radical benzyle;

$R_4$ est le triméthylène; et $R_5$ est le méthylène; ou $R_4$ et $R_5$ sont tous deux l'éthylène; étant entendu que $R_4$ et $R_5$ ont tous les deux la possibilité d'être au moins monosubstitués par un radical méthyle ou phényle;

X est un anion d'un acide minéral ou organique dans lequel un ou plusieurs hydrogènes acides ont été substitué(s) par le cation aminé; et

n peut être égal à 1, 2 ou 3 et représente le nombre d'atomes d'hydrogène salifiés.

2. Formiate de 4,4-di(t.butylperoxy)-2,2,6,6-tétraméthylpipéridine.

3. Acétate de 4,4-di(t.butylperoxy)-2,2,6,6-tétraméthylpipéridine.

4. Benzoate de 4,4-di(t.butylperoxy)-2,2,6,6-tétraméthylpipéridine.

5. Oxalate de 4,4-di(t.butylperoxy)-2,2,6,6-tétraméthylpipéridine.

6. p-toluène-sulfonate de 4,4-di(t.butylperoxy)-2,2,6,6-tétraméthylpipéridine.

7. Sulfate acide de 4,4-di(t.butylperoxy)-2,2,6,6-tétraméthylpipéridine.

8. Phosphate acide de 4,4-di(t.butylperoxy)-2,2,6,6-tétraméthylpipéridine.

9. Chlorhydrate de 4,4-di(t.butylperoxy)-2,2,6,6-tétraméthylpipéridine.

10. Sulfate acide de 4,4-di(t.butylperoxy)-1,2,2,6,6-pentaméthylpipéridine.

11. Une composition caractérisée en ce qu'elle comporte un polymère synthétique ou naturel plastomère ou élastomère et un composé ainsi que revendiqué dans l'une quelconque des revendications 1 à 10.

12. Une composition ainsi que revendiquée dans la revendication 11, caractérisée en ce que le polymère plastomère est le polyéthylène ou le polymère élastomère est un copolymère éthylène/propylène.

13. Une composition ainsi que revendiquée dans la revendication 11 ou 12, caractérisée en ce que ledit peroxyde est utilisé en tant qu'agent de réticulation en une proportion de 0,5 à 10% exprimée en poids par rapport au plastomère.

14. Une composition ainsi que revendiqué dans la revendication 13, caractérisée en ce que la quantité de peroxyde utilisée comme agent de réticulation est comprise entre 2 et 6% exprimée par rapport au plastomère.

15. Une composition ainsi que revendiquée dans la revendication 11 ou 12, caractérisée en ce que ledit peroxyde est utilisé en tant qu'agent de vulcanisation en une quantité comprise entre 0,5 et 10% exprimée par rapport à l'élastomère.

16. Une composition ainsi que revendiquée dans la revendication 15, caractérisée en ce que la quantité de peroxyde agissant en tant qu'agent de vulcanisation est comprise entre 2 et 6% exprimée par rapport à l'élastomère.

17. Une composition ainsi que revendiquée dans la revendication 12, caractérisée en ce qu'elle comprend en outre une charge et un co-agent de réticulation.

18. Une composition ainsi que revendiquée dans la revendication 12, caractérisée en ce que les

constituants, autres que ledit peroxyde de ladite composition, sont présents dans les proportions suivantes:

| | |
|---|---|
| . copolymère éthylène/propylène | 100 parties en poids |
| . noir de carbone | 50 parties en poids |
| . ZnO (ou MgO) | 5 parties en poids |
| . soufre | 0,3 parties en poids |

19. Une composition caractérisée en ce qu'elle comprend une résine thermodurcissable et un composé ainsi que revendiqué dans l'une quelconque des revendications 1 à 10.

20. Une composition ainsi que revendiquée dans la revendication 19, caractérisée en ce que la résine thermodurcissable est une résine polyester insaturée.

21. Une composition ainsi que revendiquée dans la revendication 20, caractérisée en ce que ledit peroxyde est utilisé en tant qu'agent de durcissement en une quantité comprise entre 0,1 et 5% en poids exprimée par rapport à la résine polyester insaturée.

22. Un procédé ainsi que revendiqué dans la revendication 20, caractérisé en ce qu'il comprend en outre une charge et un co-agent de réticulation.

23. Un procédé de réticulation de la composition selon la revendication 11, 12 ou 13, caractérisé en ce que la réticulation est effectuée à une température comprise entre 100 et 200°C, à une pression comprise entre 49 et 196,2 bars (50 à 200 $kg/cm^2$) pendant 5 à 60 minutes.

24. Un procédé ainsi que revendiqué dans la revendication 23, caractérisé en ce que ladite température est comprise entre 140 et 170°C et en ce que la durée est comprise entre 10 et 30 minutes.

25. Un procédé de vulcanisation de la composition selon la revendication 11, 12 ou 15, caractérisé en ce que la vulcanisation est effectuée à une température comprise entre 140 et 190°C, pendant 5 à 200 minutes.

26. Un procédé ainsi que revendiqué dans la revendication 25, caractérisé en ce que ladite température est comprise entre 150 et 170°C et la durée est de 5 à 30 minutes.

27. Un procédé de durcissement d'une composition selon la revendication 19, 20 ou 21, caractérisé en ce que le durcissement est effectué à une température comprise entre 50 et 170°C.

28. Un procédé ainsi que revendiqué dans la revendication 27, caractérisé en ce que ladite température est comprise entre 80 et 115°C.

29. L'emploi de diperoxydes selon l'une quelconque des revendications 1 à 10, en tant qu'agent de réticulation pour les plastomères, les élastomères naturels ou synthétiques et les résines polyester insaturées.